(19) Europäisches Patentamt
European Patent Office
Office européen des brevets

(11) EP 1 236 462 A1

(12) **EUROPEAN PATENT APPLICATION**

(43) Date of publication:
**04.09.2002 Bulletin 2002/36**

(21) Application number: **01110568.1**

(22) Date of filing: **30.04.2001**

(51) Int Cl.[7]: **A61K 7/48**, A61K 7/00, A61K 31/07, A61K 31/355, A61K 31/201, A61K 31/202, A61K 9/107

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.02.2001 KR 2001010460**

(71) Applicant: **Neopharm Co., Ltd.**
**Daejeon-shi, 305-333 (KR)**

(72) Inventors:
- **Park, Byeong-deog**
  **Chongju-shi, Chungchongbuk-do (KR)**
- **Youm, Jong-kyung**
  **Daejeon-shi (KR)**
- **Shim, Jae-dong**
  **Daejeon-shi (KR)**

(74) Representative: **Patentanwälte**
**Schaad, Balass, Menzl & Partner AG**
**Dufourstrasse 101**
**Postfach**
**8034 Zürich (CH)**

(54) **Multi-lamella based emulsion for stabilizing dermatologically useful ingredients and external preparation containing the same**

(57) Disclosed is a multi-lamellar emulsion for stabilizing dermatologically useful ingredients and an external preparation utilizing the same. The multi-lamellar emulsion (MLE) for stabilizing a dermatologically useful oleophilic ingredient includes: a lipid-based component comprising a lipid ingredient chosen among horny cells lipids; an oil-based component; and an emulsion-based component being a non-ionic surfactant, wherein the MLE has an oil composition represented by Equation 1:

[Equation 1]

$$\text{oil composition} = \frac{\text{content of oil-based component}}{\text{content of emulsion-based component + content of lipid-based component}} = 0.3 \sim 1.0$$

EP 1 236 462 A1

## Description

## BACKGROUND OF THE INVENTION

### 1. Field of the Invention

The present invention relates to a multi-lamellar emulsion for stabilizing dermatologically useful ingredients and an external preparation utilizing the same, and more particularly, to a multi-lamellar emulsion having an optimized oil composition for stabilizing dermatologically useful oleophilic ingredients, and an external preparation containing the dermatologically useful oleophilic ingredients.

### 2. Description of the Related Art

Dermatologically dermatologically useful oleophilic ingredients, including retinol, unsaturated fatty acids, tocopherol and derivatives thereof, fat soluble pigment, vitamin F, etc. are widely used for skin improvement and treatment. Unfortunately, these dermatologically useful oleophilic ingredients readily become unstable in an aqueous solution or under exposure to the air. Hence stabilization of the dermatologically useful oleophilic ingredients is the primary issue in regard to cosmetics and external preparations for skin improvement and treatment, many approaches for stabilizing the dermatologically useful oleophilic ingredients have been contrived.

The methods for stabilizing dermatologically useful oleophilic ingredients are largely divided into the encapsulation method and the method for utilizing the emulsified formulation. The encapsulation method contributes to stabilization of the dermatologically useful oleophilic ingredients by hardening or coating the ingredients by using collagen, alginate, agar, gelatin, chitin or chitosan derivatives to form a primary or secondary capsule and thereby protecting the ingredients against air or water. The method for utilizing the emulsified formulation includes a method of using BHT, acetic tocopherol and EDTA chelate in the simple oil in water (o/w) type or water in oil (w/o) type formulation, a method of using a w/o/w type or o/w/o type multi-lamella emulsion, and a method of delivering the ingredients in the unit of multi-lamella vesicle (MLV) using lecithin. The encapsulation method provides a relatively high stability to the dermatologically useful oleophilic ingredients but has drawbacks such as unpleasant feeling in use and a need of breaking capsules. On the other hand, the method of using the emulsified formulation provides expediency and pleasant feeling in use but poor stability.

## SUMMARY OF THE INVENTION

It is, therefore, an object of the present invention to provide a multi-lamellar emulsion having an optimized oil composition as a base for stabilizing dermatologically useful oleophilic ingredients that provides pleasant feeling in use and high stability and can be prepared in a simple way.

It is another object of the present invention to provide an external preparation for skin improvement and treatment using the multi-lamellar emulsion for stabilizing dermatologically useful oleophilic ingredients.

To achieve the above objects of the present invention, there is provided a multi-lamellar emulsion (MLE) for stabilizing a dermatologically useful oleophilic ingredient, the MLE comprising a lipid-based component, an oil-based component, and an emulsion-based component, which have predetermined oil composition that will be described later.

Preferably, the lipid-based component as used herein may include, if not specifically limited to, a pseudoceramide, cholesterol and derivatives thereof, higher fatty acid, higher fatty alcohol, or any mixture thereof having the following formula 1 or 2. According to the present invention, the lipid-based component may include any lipid ingredient between horny cells.

[Formula 1]

$R_1$-CO

|

$R_2$-CH-CONH$(CH_2)_2$OH

wherein $R_1$ and $R_2$ are straight-chain or branched alkyl having 6 to 22 carbon atoms,

[Formula 2]

$$\begin{array}{l} R_1\text{-CHOH} \\ | \\ R_2\text{-CH-CONH}(CH_2)_2OH \end{array}$$

wherein $R_1$ and $R_2$ are straight-chain or branched alkyl having 6 to 22 carbon atoms.

The oil-based component as used herein may include, if not specifically limited to, either saturated hydrocarbon-based oils or unsaturated hydrocarbon-based oils or any mixture thereof. The saturated hydrocarbon-based oils may include mineral oil or synthetic oil, e.g., liquid paraffin, squalan, vaseline, paraffin wax, or other branched hydrocarbon-based oils. The unsaturated hydrocarbon-based oils may include natural oils such as animal oils and vegetable oils, which are all usable herein. The present invention may use a single type of oil and, more preferably, a mixture of different oils. The reason of this is as follows. In preparing the MLE using the single type of oil, the saturated hydrocarbon-based oils are insufficient to form the MLE but favorable to stabilization of the dermatologically useful oleophilic ingredient, while natural oils in the form of triglyceride among the unsaturated hydrocarbon-based oils are favorable to the formation of the MLE but inferior to the saturated hydrocarbon-based oils in stabilizing the dermatologically useful oleophilic ingredient. For that reason, using a mixture of different oils is preferable in both formation of the MLE and stabilization of the dermatologically useful oleophilic ingredient.

The emulsion-based component as used herein is preferably a non-ionic surfactant, examples of which may include, if not specifically limited to, polyoxyethylene fatty acid ester, polyoxyethylene glycerin fatty acid ester, polyoxyethylene sorbitan fatty acid ester, glycerin fatty acid ester, or any mixture thereof.

The MLE of the present invention includes water as well as the above-mentioned components, and additionally a thickener.

The predetermined oil composition optimized in the present invention satisfies the following Equation 1, in which the value of the content is based on the weight:

[Equation 1]

$$\text{oil composition} = \frac{\text{content of oil-based component}}{\text{content of emulsion-based component + content of lipid-based component}} = 0.3 \sim 1.0$$

An optimal oil composition in the MLE or MLE-based external preparations according to the present invention is in the range of 0.3 to 1.0, more preferably, 0.5 to 0.8 in the aspect of stabilization of the dermatologically useful oleophilic ingredient.

The external preparations of the present invention comprise the MLE of the present invention and dermatologically useful oleophilic ingredient incorporated therein. Preferred examples of the dermatologically useful oleophilic ingredient may include, if not specifically limited to, retinol and derivatives thereof, unsaturated fatty acids and derivatives thereof, vitamin F, tocopherol and derivatives thereof, and fat soluble pigments. Examples of the retinol derivatives may include, if not specifically limited to, retinic acid, retinyl aldehyde, retinyl acetate, retinyl propionate, retinol palmitate, retinyl oleate, or retinyl linolate. Examples of the tocopherol derivatives may include, if not specifically limited to, tocopheryl ester such as tocopheryl acetate or tocopheryl linolate.

The added amount of the dermatologically useful oleophilic ingredient in the present invention is, based on the total weight of the external preparation, 0.001 to 30 wt.%, preferably 0.1 to 20 wt.%, and more preferably 0.2 to 10 wt.%.

Hereinafter, a description will be given as to a method for preparing an MLE having a predetermined oil composition according to the present invention.

The MLE preparation method includes melting pseudo-ceramide, cholesterol and derivatives thereof, higher fatty acid, higher fatty alcohol, polyalcohol or any mixture thereof together with general oils and a non-ionic surfactant, uniformly mixing these components, adding a warm purified water to the mixture to cause phase inversion emulsification, adding a thickener immediately after the emulsification and uniformly mixing these components, and cooling the resulting mixture at the room temperature. The added amount of the purified water is, if not specifically limited to, 50 to 95 wt.%, and preferably 65 to 85 wt.% based on the total weight of the MLE.

On the other hand, optimization of the oil composition of the MLE involves drawing up a three-phase diagram

shown in FIG. 1; preparing a lipid phase comprising pseudo-ceramide, cholesterol, higher fatty acid, higher fatty alcohol, or any mixture thereof, and an emulsion phase comprising a non-ionic surfactant to form a lipid-emulsion system in which the MLE will be formed; and adding different oils to regulate the oil composition.

## BRIEF DESCRIPTION OF THE DRAWINGS

FIG. 1 is a three-phase (emulsion-oil-lipid) diagram for preparing a multi-lamella emulsion having an optimal oil composition according to the present invention; and

FIGS. 2a and 2b are fluorescence microscopic pictures showing the lamella liquid crystalline structure of an external preparation using the multi-lamella emulsion according to the present invention, in which FIGS. 2a and 2b show the excellent ageing stability of the lamella liquid crystalline structure after four weeks at 25 °C and 45 °C, respectively.

## DETAILED DESCRIPTION OF THE PREFERRED EMBODIMENT

Hereinafter, the present invention will be described in detail by way of the following examples and experimental examples, which are not intended to limit the scope of the present invention.

<u>Examples 1 to 8 and Comparative Examples 1 to 4</u>

Pseudo-ceramide PC-9S, cholesterol, higher fatty acid, non-ionic surfactant, higher fatty alcohol, polyalcohol, thickener and purified water were mixed with the composition presented in Table 1 to prepare a multi-lamella emulsion (MLE). Retinol palmitate was used as an indicator of the dermatologically useful oleophilic ingredient. Various MLE's were prepared depending on the type of the emulsifier (in Examples 1 to 4) or the oil composition (in Examples 5 to 8). The MLE's of Comparative Examples 1 and 2 didn't contain cholesterol or fatty acid as an intercellular lipid ingredient, the MLE of Comparative Example 3 contained no oil (in Comparative Example 3), and the MLE of Comparative Example 4 was scarcely formed due to the excessively high oil composition.

The pseudo-ceramide PC-9S had the above-mentioned formula (1), in which $R_1$ is $-C_{15}H_{31}$ or $C_{17}H_{35}$; and $R_2$ is $-C_{14}H_{29}$ or $C_{16}H_{33}$. The thickener was carboxy vinyl polymer.

Table 1

| Ingredient | A1 | A2 | A3 | A4 | A5 | A6 | A7 | A8 | B1 | B2 | B3 | B4 |
|---|---|---|---|---|---|---|---|---|---|---|---|---|
| Pseudo-ceramide | 0.5 | 0.8 | 1.0 | 0.4 | 0.5 | 0.6 | 0.8 | 1.0 | - | - | 1.0 | 1.0 |
| Natural ceramide (III-B) | - | - | - | - | - | - | - | - | 0.3 | - | - | - |
| Cholesterol | 0.25 | 0.4 | 0.1 | 0.15 | 0.15 | 0.2 | 0.3 | 0.3 | - | - | 0.3 | 0.3 |
| Stearic acid | 1.0 | 2.8 | 3.5 | 1.0 | 1.2 | 1.5 | 1.8 | 2.0 | - | 1.6 | 2.0 | 2.0 |
| Cetostearylacohol | 2.0 | 2.0 | 1.6 | 2.0 | 2.5 | 3.0 | 3.5 | 4.0 | 3.5 | 1.4 | 4.0 | 4.0 |
| POE mono-stearate | 3.0 | - | - | - | - | - | - | - | - | - | - | - |
| POE sorbitan mono-stearate | - | - | 2.2 | - | - | - | - | - | - | - | - | - |
| Sorbitan mono-stearate | - | - | 0.5 | - | - - | - |  | - | - | - | - | - |
| POE glyceryl mono-stearate | - | 3.0 | - | 2.0 | 2.5 | 3.0 | 3.2 | 4.0 | 2.0 | 2.0 | 4.0 | 4.0 |
| Glyceryl mono-stearate | 2.0 | 1.0 | 1.2 | 1.0 | 1.2 | 1.5 | 1.8 | 2.0 | 1.0 | 1.0 | 2.0 | 2.0 |
| Oils Olive oil Liquid paraffin Vaseline Paraffin Wax | 5.0 | 5.0 | 5.0 | 4.0 | 3.0 | 5.0 | 8.0 | 13.0 | 5.0 | 4.0 | 0.0 | 20.0 |
| 1,3-butylene glycol | 5.0 | 5.0 | 5.0 | 5.0 | 5.0 | 5.0 | 5.0 | 5.0 | 5.0 | 5.0 | 5.0 | 5.0 |
| Retinol palmitate | 0.3 | 0.3 | 0.3 | 0.3 | 0.3 | 0.3 | 0.3 | 0.3 | 0.3 | 0.3 | 0.3 | 0,3 |
| Thickener | 0.2 | 0.2 | 0.2 | 0.2 | 0.2 | 0.2 | 0.2 | 0.2 | 0.2 | 0.2 | 0.2 | 0.2 |
| Purified water | dregs | dregs | dregs | dregs | dregs | dregs | dregs | dregs | dregs | dregs | dregs | dregs |
| Oil composition | 0.57 | 0.5 | 0.5 | 0.61 | 0.37 | 0.55 | 0.70 | 0.99 | 0.75 | 0.67 | 0 | 1.50 |

Note) A: Example
B: Comparative Example
Oil composition = content of oil-based component / (content of emulsion-based component + content of lipid-based component)

Experimental Example 1: Thermal Stability Test

A four-week thermal stability test was performed on the MLE's prepared in Examples 1 to 8 and Comparative Examples 1 to 4 in order to measure the stability of retinol palmitate. After 4 weeks at 25 °C and 45 °C, high-performance liquid chromatography (HPLC) was utilized to analyze stable retinol palmitate. The results are presented in Table 2.

Table 2

| | A1 | | A2 | | A3 | | A4 | | B1 | | B2 | |
|---|---|---|---|---|---|---|---|---|---|---|---|---|
| | 25°C | 45°C | 25°C | 45°C | 25°C | 45°C | 25°C | 45°C | 25°C | 45°C | 25°C | 45°C |
| After 2 weeks (%) | 95.3 | 68.9 | 94.2 | 71.2 | 91.2 | 72.3 | 93.3 | 68.3 | 72.3 | 60.2 | 68.9 | 58.6 |
| After 4 weeks (%) | 92.5 | 59.5 | 91.2 | 67.4 | 89.3 | 58.2 | 88.9 | 59.3 | 54.3 | 45.3 | 53.2 | 42.5 |
| Temp / Term | A5 | | A6 | | A7 | | A8 | | B3 | | B4 | |
| | 25°C | 45°C | 25°C | 45°C | 25°C | 45°C | 25°C | 45°C | 25°C | 45°C | 25°C | 45°C |
| After 2 weeks (%) | 99.8 | 73.0 | 98.9 | 75.9 | 95.9 | 82.3 | 94.9 | 77.2 | 73.1 | 71.2 | 89.7 | 62.3 |
| After 4 weeks (%) | 97.2 | 60.7 | 95.4 | 65.8 | 94.6 | 67.3 | 93.0 | 66.3 | 64.3 | 23.9 | 74.2 | 48.2 |

Note) A: Example

B: Comparative Example

As is apparent from the results of Table 2 (Examples 5 to 8), the stability of retinol palmitate was increased with an increase in the oil composition. However, for Examples 7 and 8, the stability of retinol palmitate was lower at the oil composition of about 1.0 than at the oil composition of about 0.7 (Example 7). This means that there is an optimal oil composition in the MLE of the present invention. Also, as is seen from the results of Examples 1 to 4 and Comparative Examples 1 and 2, the stability of retinol palmitate was low without a proper mixture of horny intercellular lipid gradients, cholesterol and fatty acid even though the oil composition was within the range of 0.3 to 1.0.

Experimental Example 2: Stability Test of External MLE Preparation

To measure the stability of the lamella liquid crystalline structure of the external MLE preparation containing retinol palmitate, the preparation of Example 3 was placed at 25 °C or 45 °C for 4 weeks and observed with a polarized microscope (Optiphotpol-2, Nikon Company) whether the maltese cross peculiar to the MLE appeared. The results are presented in FIG. 2.

As is apparent from FIG. 2, the lamella liquid crystalline structure of the MLE according to the present invention

was stable after 4 weeks at 45 °C (in FIG. 2b) as well as after 4 weeks at 25 °C (in FIG. 2a). Such a high stability contributes to effective stabilization of various dermatologically useful oleophilic ingredients such as retinol palmitate.

Experimental Example 3: Skin Stimulation Test

To measure the skin stimulation ability of the external MLE preparation of the present invention, 30 healthy adult persons had the upper arm onto which Haye's chambers stained with a predetermined amount (about 2.0 g), which is used for the patch test, of the individual preparations of Example 6 and Comparative Examples 1 and 4 were applied for 48 hours. After an elapse of 30 minutes to one hour since the Haye's chambers were removed, the change of the complexion was observed with unaided eyes for the primary finding, and again after more 48 hours for the secondary finding.

As is apparent from the results in Table 3, the preparation of Example 6 less stimulated the skin than those of Comparative Examples 1 and 4.

Table 3

| Sample | Number of Testees | Results | | | | | | | | | |
|---|---|---|---|---|---|---|---|---|---|---|---|
| | | Non | | Uncertain | | Slightly | | Yes | | Severe | |
| | | I | II | I | II | I | II | I | II | I | II |
| Example 6 | 30 | 30 | 30 | | | | | | | | |
| Comparative Example1 | 30 | 25 | 30 | 5 | | | | | | | |
| Comparative Example 4 | 30 | 24 | 28 | 1 | 4 | 5 | 2 | | | | |

As described above, the external preparation using the MLE having an optimal oil composition according to the present invention for stabilizing dermatologically useful oleophilic ingredients is incorporated with the dermatologically useful oleophilic ingredients which are effectively stabilized, which exhibits excellent appetence to the skin, thereby providing various external preparations for skin improvement and treatment because it hardly stimulates the skin.

## Claims

**1.** A multi-lamellar emulsion (MLE) for stabilizing a dermatologically useful oleophilic ingredient, the MLE comprising:

a lipid-based component comprising a lipid ingredient between horny cells;
an oil-based component; and
an emulsion-based component being a non-ionic surfactant.

wherein the MLE has an oil composition represented by Equation 1:

[Equation 1]

$$\text{oil composition} = \frac{\text{content of oil-based component}}{\text{content of emulsion-based component + content of lipid-based component}} = 0.3 \sim 1.0$$

**2.** The MLE as claimed in claim 1, wherein the lipid-based component includes a pseudo-ceramide, cholesterol and derivatives thereof, higher fatty acid, higher fatty alcohol, or any mixture thereof having the formula 1 or 2:

[Formula 1]

$R_1$-CO

|

$R_2$-CH-CONH$(CH_2)_2$OH

wherein $R_1$ and $R_2$ are straight-chain or branched alkyl having 6 to 22 carbon atoms,

[Formula 2]

$R_1$-CHOH

|

$R_2$-CH-CONH$(CH_2)_2$OH

wherein $R_1$ and $R_2$ are straight-chain or branched alkyl having 6 to 22 carbon atoms.

**3.** The MLE as claimed in claim 2, wherein the oil-based component is selected from the group consisting of saturated hydrocarbon-based oils, unsaturated hydrocarbon-based oils, or saturated and unsaturated hydrocarbon-based oils, wherein the emulsion-based component is selected from the group consisting of polyoxyethylene fatty acid ester, polyoxyethylene glycerin fatty acid ester, polyoxyethylene sorbitan fatty acid ester, glycerin fatty acid ester, or any mixture thereof.

**4.** The MLE as claimed in any one of claims 1, 2 and 3, wherein the oil composition is in the range from 0.5 to 0.8.

**5.** The MLE as claimed in claim 2, wherein the lipid-based component includes a pseudo-ceramide having the formula 1 or 2, wherein $R_1$ is —$C_{15}H_{31}$ or $C_{17}H_{35}$; and $R_2$ is -$C_{14}H_{29}$ or $C_{16}H_{33}$.

**6.** The MLE as claimed in any one of claims 1, 2 and 3, or claim 5, wherein purified water is contained in an amount of 60 to 95 wt.% based on the total weight of the MLE.

**7.** The MLE as claimed in any one of claims 1, 2 and 3, or claim 5, wherein the dermatologically useful oleophilic ingredient is at least one compound selected from the group consisting of retinol and derivatives thereof, unsaturated fatty acids and derivatives thereof, vitamin F, tocopherol and derivatives thereof, and fat soluble pigments.

**8.** The MLE as claimed in any one of claims 1, 2 and 3, or claim 5, wherein the dermatologically useful oleophilic ingredient according to claim 6 is contained in an amount of 0.001 to 30 wt.% based on the total weight of the dermatologically useful oleophilic ingredient.

FIG. 1

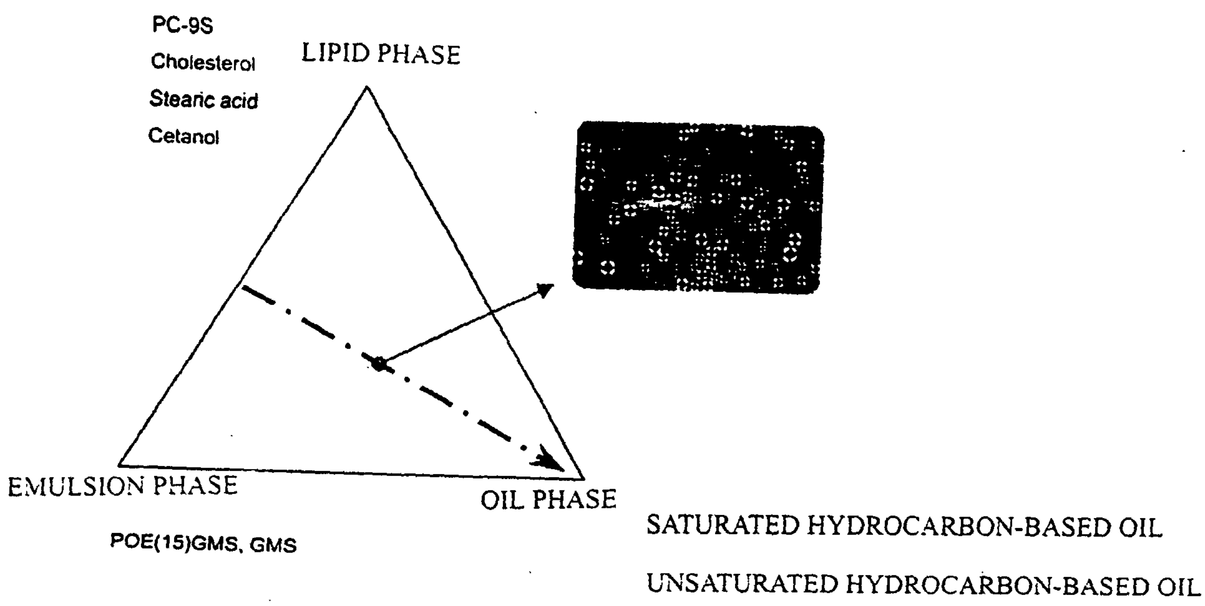
PC-9S
Cholesterol
Stearic acid
Cetanol
LIPID PHASE
EMULSION PHASE
OIL PHASE
POE(15)GMS, GMS
SATURATED HYDROCARBON-BASED OIL
UNSATURATED HYDROCARBON-BASED OIL

FIG. 2A

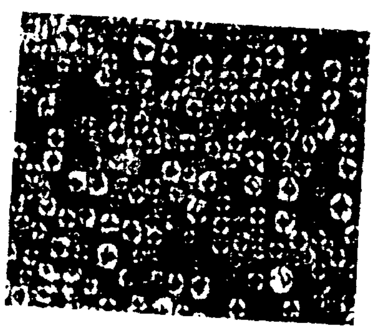

FIG. 2B

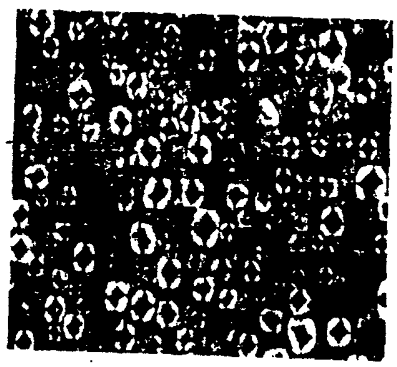

European Patent Office

# EUROPEAN SEARCH REPORT

Application Number
EP 01 11 0568

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 875 232 A (TAKASAGO PERFUMERY CO LTD) 4 November 1998 (1998-11-04)<br>* page 2, line 1-12,42-54 *<br>* page 3, line 22 - page 4, line 49 *<br>* page 5, line 9-12 *<br>* page 7, line 34-44 *<br>* page 8, line 48 - page 9, line 3 *<br>* page 9, line 37,38,45,46 *<br>* page 10, line 3,4,11,12,21,22 *<br>* page 10, line 49 - page 11, line 5 *<br>* page 13, line 26 - page 14, line 11 *<br>* claims 1,2,9,10,12-14 *<br>--- | 1-4,6-8 | A61K7/48<br>A61K7/00<br>A61K31/07<br>A61K31/355<br>A61K31/201<br>A61K31/202<br>A61K9/107 |
| X | US 5 980 917 A (KIM JUNG SU ET AL) 9 November 1999 (1999-11-09)<br>* column 2, line 19-32 *<br>* column 3, line 5 - column 4, line 11 *<br>* examples 1-3 *<br>* column 7, line 39-45 *<br>* claims 1-3 *<br>--- | 1-3,6-8 | |
| A | WO 98 21176 A (BAIK IN SOB ;LEE JONG GI (KR); LEE MYUNG JIN (KR); KIM YOON (KR);) 22 May 1998 (1998-05-22)<br>* page 1, line 4-8 *<br>* page 5, line 14 - page 6, line 14 *<br>* table 1 *<br>* page 15, line 6-13 *<br>* examples 13,14,19 *<br>* tables 2,7 *<br>--- | 1-8 | TECHNICAL FIELDS SEARCHED (Int.Cl.7)<br>A61K |
| A | US 4 999 348 A (CIOCA GHEORGHE ET AL) 12 March 1991 (1991-03-12)<br>* column 1, line 13-18 *<br>* column 2, line 7-24 *<br>* column 3, line 18-56 *<br>* claims 1-4 *<br>---<br>-/-- | 1-8 | |

The present search report has been drawn up for all claims

| Place of search | Date of completion of the search | Examiner |
|---|---|---|
| THE HAGUE | 9 August 2001 | Bazzanini, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 11 0568

| Category | Citation of document with indication, where appropriate, of relevant passages | Relevant to claim | CLASSIFICATION OF THE APPLICATION (Int.Cl.7) |
|---|---|---|---|
| A | WO 88 06882 A (MICRO VESICULAR SYSTEMS) 22 September 1988 (1988-09-22)<br>* page 1, line 24 - page 2, line 3 *<br>* page 5, line 26-31 *<br>* page 8, line 5-27 *<br>* page 10, line 24-27 *<br>* page 11, line 1-7 *<br>* page 12, line 4-14 *<br>* page 13, line 3-6,13-17 *<br>* page 14, line 4-21 *<br>* claims 9-12 *<br>----- | 1-8 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 9 August 2001 | Bazzanini, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 11 0568

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2001

| Patent document cited in search report | Publication date | Patent family member(s) | Publication date |
|---|---|---|---|
| EP 0875232 A | 04-11-1998 | JP 11012118 A | 19-01-1999 |
| | | US 5916578 A | 29-06-1999 |
| US 5980917 A | 09-11-1999 | KR 258674 B | 01-07-2000 |
| | | JP 11079929 A | 23-03-1999 |
| WO 9821176 A | 22-05-1998 | US 6221371 B | 24-04-2001 |
| US 4999348 A | 12-03-1991 | CA 1333567 A | 20-12-1994 |
| WO 8806882 A | 22-09-1988 | US 4917951 A | 17-04-1990 |
| | | AT 71292 T | 15-01-1992 |
| | | AT 71289 T | 15-01-1992 |
| | | AT 69723 T | 15-12-1991 |
| | | AU 605581 B | 17-01-1991 |
| | | AU 1541688 A | 10-10-1988 |
| | | AU 603659 B | 22-11-1990 |
| | | AU 1543888 A | 10-10-1988 |
| | | AU 603447 B | 15-11-1990 |
| | | AU 1683688 A | 10-10-1988 |
| | | BR 8807410 A | 10-04-1990 |
| | | CA 1289420 A | 24-09-1991 |
| | | CA 1311415 A | 15-12-1992 |
| | | CA 1289419 A | 24-09-1991 |
| | | DE 3866544 A | 09-01-1992 |
| | | DE 3866544 D | 09-01-1992 |
| | | DE 3867635 A | 20-02-1992 |
| | | DE 3867635 D | 20-02-1992 |
| | | DE 3867637 A | 20-02-1992 |
| | | DE 3867637 D | 20-02-1992 |
| | | DK 633188 A | 11-11-1988 |
| | | DK 633388 A | 11-11-1988 |
| | | EP 0349579 A | 10-01-1990 |
| | | EP 0352282 A | 31-01-1990 |
| | | EP 0349593 A | 10-01-1990 |
| | | HU 53279 A | 28-10-1990 |
| | | JP 2502794 T | 06-09-1990 |
| | | JP 2617346 B | 04-06-1997 |
| | | JP 2502094 T | 12-07-1990 |
| | | JP 2589173 B | 12-03-1997 |
| | | JP 6000193 B | 05-01-1994 |
| | | JP 2503646 T | 01-11-1990 |
| | | KR 9609647 B | 23-07-1996 |
| | | NO 885032 A | 11-11-1988 |
| | | NO 885036 A | 11-01-1989 |
| | | NZ 223843 A | 26-04-1990 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 01 11 0568

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2001

| Patent document cited in search report | Publication date | Patent family member(s) | Publication date |
|---|---|---|---|
| WO 8806882 A | | US 4855090 A | 08-08-1989 |
| | | WO 8806881 A | 22-09-1988 |
| | | WO 8806883 A | 22-09-1988 |
| | | US 4911928 A | 27-03-1990 |
| | | US 5474848 A | 12-12-1995 |
| | | US 4942038 A | 17-07-1990 |
| | | US 5023086 A | 11-06-1991 |
| | | US 5000960 A | 19-03-1991 |
| | | US 5628936 A | 13-05-1997 |
| | | US 5219538 A | 15-06-1993 |
| | | US 5234767 A | 10-08-1993 |
| | | ZA 8801763 A | 12-09-1988 |
| | | US 5147723 A | 15-09-1992 |

EPO FORM P0459

For more details about this annex : see Official Journal of the European Patent Office, No. 12/82